# EUROPEAN PATENT APPLICATION

(11) **EP 0 614 678 A1**
(43) Date of publication of application: **14.09.1994**
(21) Application number: 94101823.6
(22) Date of filing: 07.02.1994
(51) Int. Cl.: A61N 1/372, A61B 5/044, A61B 5/042

(54) **Apparatus for measuring electrical activity in the heart**

(30) Priority: 12.03.1993 SE 9300825
(71) Applicant: Siemens Elema AB, S-171 95 Solna 1 (SE)
(72) Inventor: Laurent, Estan, S-610 24 Vikbolandet (SE); Magnusson, Monica, S-181 61 Lidingö (SE)

(57) **Abstract**

An apparatus for measuring electrical activity in the heart comprises an input amplifier unit (36) with a plurality of inputs to which poles of electrodes introduced into the patient are intended to be connected. The input amplifier unit comprises sensing means (48) for sensing the impedance across the amplifier inputs after connection of the electrodes in order to identify the poles which are in contact with the patient's heart, and a display (24) is connected to the amplifier unit to graphically indicate which electrode poles are connected and which are in contact with the heart.

## Description

This invention relates to an apparatus for measuring electrical activity in the heart, comprising an input amplifier unit with a plurality of inputs to which poles of electrodes introduced into the patient are intended to be connected.

Devices for processing physiological signals are previously known from e.g. US-A-3,994,286 and US-A-4,023,565. These devices are intended for use in ECG and EEG recordings and are specially designed to suppress certain noise signals and enable simple realization.

From US-A-4,213,465, an electroencephalograph is known with switches arranged in a pattern reproducing the head. The measurement electrodes are attached with these switches to input channels, the position of the switches in the main representation of the head corresponding to the positions of the electrodes on the patient's head. In this manner, the position of the switches in the rendition of the head provides the operator with a good overview of electrode placement.

The object of the present invention is to achieve a device for measuring electrical activity in the heart of a patient, the way the electrode poles are connected to the heart being graphically depicted.

This object is achieved with an apparatus of the above-described kind with the features set forth in claim 1.

Thus, the apparatus according to the invention is intended for measuring electrical activity in the heart, i.e. IECG, both spontaneous activity and any activity triggered by the apparatus. The apparatus automatically senses which electrode poles are in contact with the heart. This is appropriately depicted on a graphical screen. This graphical screen thereby provides the operator with a good overview of the coupling of the electrode poles to the amplifiers, shows whether an electrode is connected to the apparatus and indicates whether there is any break in any of the electrode poles.

According to an advantageous embodiment of the apparatus according to the invention, an A/D converter's output terminal is connected, via an isolation interface, to a microprocessor for signal processing and a following control processor for controlling the graphical representation of the poles on the display. The isolation interface isolates the patient from other electrical equipment.

According to another advantageous further development of the apparatus according to the invention, a potential sensed by a predetermined electrode pole serves as a reference voltage for all input amplifiers in the input amplifier unit. In this manner, the voltage at the first electrode pole connected, for example, can be employed as a reference for the amplifiers instead of isolated ground.

According to yet another advantageous embodiment of the apparatus according to the invention, a heart stimulator is connectable to an optional electrode pole via a switching unit. Thus, the apparatus according to the invention advantageously contains a built-in stimulator to save space in the examination room, which usually contains a large amount of equipment already, and so the same programming can be used as for the measurement function. Great flexibility is achieved in stimulation when the heart stimulator is connectable to optional electrode poles.

According to another advantageous embodiment of the apparatus according to the invention, shunt diodes are arranged on the input amplifiers' inputs to protect them against high defibrillation voltages.

One exemplified embodiment of the apparatus according to the invention will now be described in greater detail, referring to attached drawings in which
FIG. 1 illustrates the use of a system for recording an IECG with the apparatus according to the invention;
FIG. 2 is a block diagram of the equipment for recording IECG's with the system shown in FIG. 1; and
FIG. 3 is a circuit diagram showing part of the input amplifier unit more in detail.

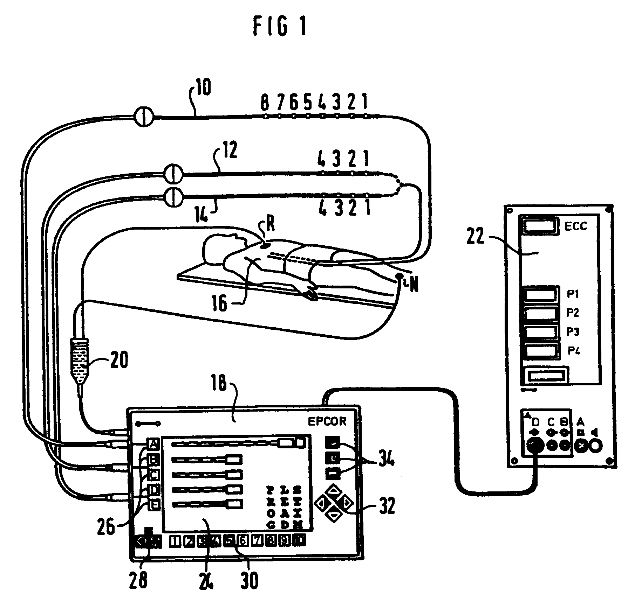

In FIG. 1 is schematically shown three electrode catheters 10, 12, 14 implanted in a patient 16 for measuring electrical activity in the heart, i.e. for recording IECG's. The electrode 10 has 8 poles, 1 - 8, and the electrodes 12 and 14 have each 4 poles, 1 - 4. A reference electrode R and a neutral electrode N are also attached to the patient 16. The reference electrode R is only employed in unipolar measurements.

The electrode catheters 10, 12, 14, the reference electrode R and the neutral electrode N are connected by appropriate cables to the measurement equipment 18. Cables from the reference electrode R and the neutral electrode N are collected in a junction box 20.

Measurement equipment 18 is further connected to the input panel 22 on a programmable recorder for recording measured events. Since this device is not a part of the invention, it will not be described in detail.

The measurement equipment 18 comprises a display 24 on which the electrodes 10, 12, 14 with their poles 1 - 8 and 1 - 4 respectively can be graphically represented. Each connected electrode 10, 12, 14 is schematically shown on the screen, and the representation of the electrodes 10, 12, 14 shows whether or not each pole is in contact with the heart. Thus, the screen representation provides the operator with clear information on the electrodes connected to the measurement equipment 18 and whether the poles are in contact with the heart. So the operator can immediately see whether there is an interruption in any of the electrode poles.

The screen 24 is suitably background lit, since the level of lighting in examination rooms is often low.

It would be advantageous if the measurement equipment's enclosure also incorporated a built-in heart stimulator which can be connected to optional electrode poles to deliver stimulation pulses to the heart.

The front panel of the measurement equipment 18 further has different operating buttons 26, 28, 30, 32 and 34 for controlling the operation of the equipment, i.e. for e.g. connecting the desired electrode poles, selecting different measurement and stimulation functions etc.

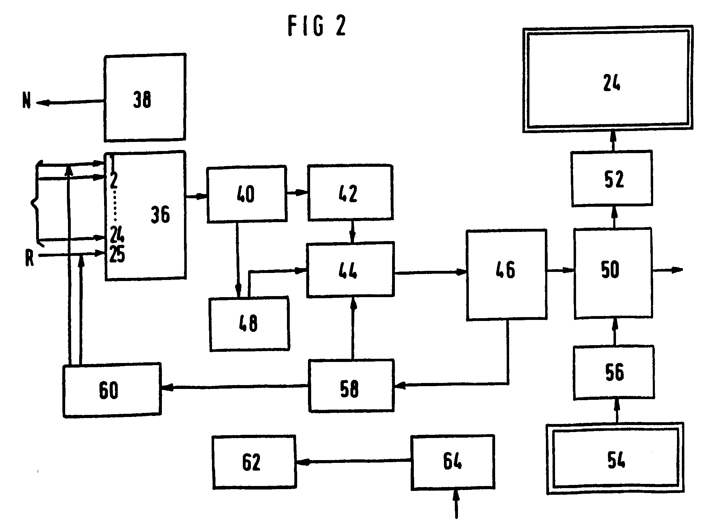

FIG. 2 is a block diagram of the measurement equipment 18 in FIG. 1. A total of 24 electrode poles, distributed among five catheters, can be connected to the input amplifier unit 36. There is further an input terminal for the reference electrode R. The equipment also comprises a connection 38 for the neutral electrode N.

A multiplexor unit 40, through which all electrode poles are multiplexed to an A/D converter 42, follows the input amplifier unit 36. The A/D-converted signals are transmitted, via an isolation interface and a transmitter 44, to a microprocessor 46 for signal processing. The isolation interface isolates the patient from following electrical equipment.

A connection detector 48, which delivers information to the transmitter 44 on the electrode poles connected, said information being forwarded on to the signal processor 46, is also connected to the multiplexor 40.

After the signal processor 46, a control processor 50 follows, which controls the display 24 via an interface circuit 52 so the coupling of the electrode poles is depicted on the display, as described above. The control processor 50 is also connected to the above-described printer input panel.

The operating keys 26, 28, 30, 32, 34 shown in FIG. 1 are represented in FIG. 2 by the keyboard 54 which is connected, via a decoder 56, to the control processor 50 for manual control of the same.

The signal processor 46 is further connected, via a receiver 58, to a heart stimulator 60 which is connectable, through a stimulation multiplexor in the form of a switching unit, to optional electrode poles. In FIG. 2 is shown the stimulator 60 connected between the electrode pole 1 and the reference pole R for emission of stimulation pulses between these electrodes.

The stimulator 60 suitable has two mutually synchronised outputs permitting synchronized stimulation in the heart's atrium and ventricle.

The equipment further comprises a requisite power supply 62, 64.

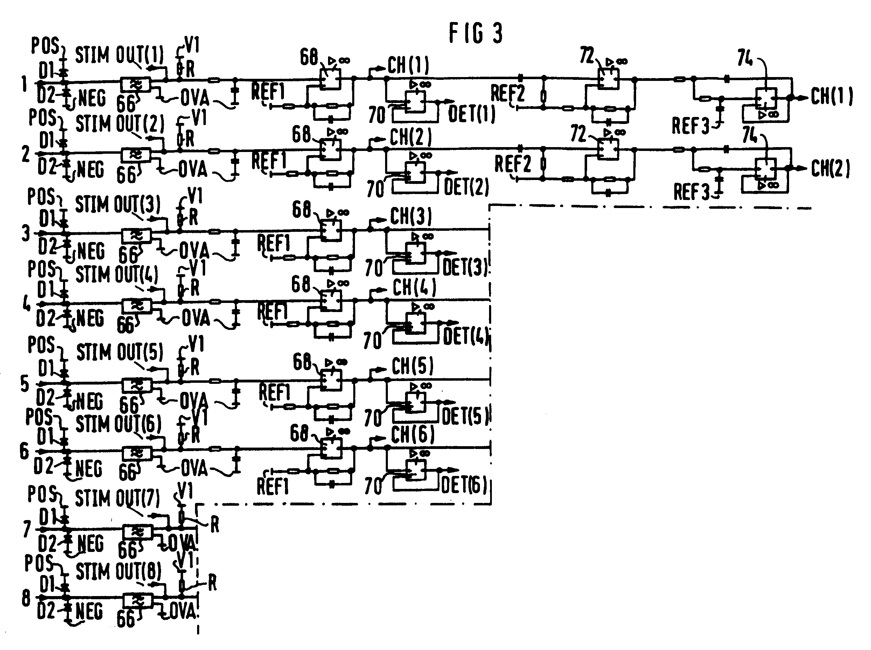

FIG. 3 is a circuit diagram of a part of the input amplifier unit 36 with eight inputs for electrode poles.

A so - called EMI filter 66 is provided at each input terminal to protect against external interference, followed by a plurality of essentially identical amplifier stages 68, 70, 72, 74 for successive amplification of the signal before it is sent to the multiplexor 40.

When input 1 is not connected to an electrode pole in contact with the heart, i.e. when this input is open and is passing no current, the voltage V₁ causes the input to go to a high level via the resistor R. This high level signal is amplified in the amplifier 68, delivered from the amplifier 70 at DET(1) for supply to the connection detector 48 and corresponding indication on the screen 24. The same signal, although further amplified in the amplifiers 72, 74, is also fed through the multiplexor 40 to the A/D converter 42 at CHANNEL(1) in the FIGURE.

When the electrode pole connected to input 1 is in contact with the heart, the input is lowered to a low level (zero level), and the corresponding low signal is supplied to the detector 48 and the A/D converter 42.

In this manner, an automatic sensing of the poles connected to the heart and the corresponding screen indications are achieved.

A voltage can be collected at CH(1) which is appropriate for use as reference voltage REF(1). The same reference voltage is then used for all input channels. The voltage CH, obtained from the first electrode pole connected, is appropriately selected.

The heart stimulator 60 is connectable, via a stimulation multiplexor, to optional electrode poles via the STIM OUT terminals.

Lead diodes D1 and D2 are provided at each input to protect the amplifiers against high defibrillation voltages. Here, the requisite resistance is obtained in the patient's body between the externally applied patch and the heart.

## Claims

1. An apparatus for measuring electrical activity in the heart, comprising an input amplifier unit (36) with a plurality of inputs to which poles of electrodes (10, 12, 14) introduced into the patient (16) are intended to be connected, characterized in that the input amplifier unit (36) comprises sensing means (V₁, R, 48) for sensing the impedance across the amplifier inputs after connection of the electrodes (10, 12, 14) in order to identify the poles which are in contact with the patient's (16) heart, and in that a display (24) is connected to the amplifier unit to graphically indicate which electrode poles are connected and which are in contact with the heart.

2. A apparatus of claim 1, characterized in that the sensing means comprise a voltage source (V₁) which is connected, via a resistor (R), to each amplifier input, so the current through the resistor and, thus, the voltage at the respective input varies according to the impedance sensed out from the input.

3. An apparatus of claim 1 or 2, characterized in that the input amplifier unit (36) is connected to a multiplexor (40) for multiplexing all inputs to an A/D converter (42) after the multiplexor.

4. An apparatus of any of claims 1 - 3, characterized in that the A/D converter's (42) output is connected to a microprocessor (46), via an isolation interface, for signal processing and to a following control processor (50) for controlling the graphical representation of the poles on the display (24).

5. An apparatus of any of claims 1 - 4, characterized in that the potential sensed at a predetermined electrode pole serves as a reference voltage for all input amplifiers in the input amplifier unit (36).

6. An apparatus of any of claims 1 - 5, characterized in that a heart stimulator (60) is connectable to any optional electrode pole via a switching unit.

7. An apparatus of claim 6, characterized in that shunt diodes (D1, D2) are arranged on the inputs to the input amplifiers to protect the amplifiers of the input amplifier unit (36) against high defibrillation voltages.

8. An apparatus of any of claims 1 - 7, characterized in that the electrodes (10, 12, 14) are multipolar.
